# EUROPEAN PATENT APPLICATION

(11) **EP 4 386 077 A1**
(43) Date of publication of application: **19.06.2024**
(21) Application number: 23195795.2
(22) Date of filing: 06.09.2023
(51) Int. Cl.: C12M 1/00, D06N 3/04, D06N 3/18

(54) **CONTAINER COMPRISING A COMPOSITE FILM**

(30) Priority: 16.09.2022 US 202263407239 P
(71) Applicant: Solvay Specialty Polymers USA, LLC, Alpharetta, Georgia 30005-3914 (US)
(72) Inventor: CHEN, Nan, Alpharetta (GA), 30005 (US); WILLIAMS, Lewis Karl, Alpharetta (GA), 30004 (US); GUNTHER, Val Glade, ALPHARETTA (GA), 30005 (US)
(74) Representative: Senninger, Thierry

(57) **Abstract**

The invention relates to a container comprising at least one composite film (CF) wherein the composite film (CF) comprises a fiber fabric (F) in between two layers (L1) and (L2) made of or comprising at least one fluoropolymer (FP) comprising recurring units (R_{TFE}) of formula -(CF₂-CF₂)-.

## Description

### Technical field

This present invention pertains to a container for storing high value products and having an inner wall made of or comprising a composite film.

### Background art

In many industries, there is a need for storing a high value product in a resistant container without any contamination of the compound by molecules migrating from the wall which is in direct contact with the product. For instance, in the field of semiconductors, products of very high purity need to be stored without any contamination to retain the high purity. These products such as H₂O₂ are sometimes very corrosive and the container should therefore be chemically resistant.

In the pharmaceutical industry, biological products need also to be stored safely without any contamination. For instance, the modern pharmaceutical bioprocessing industry often requires the use of a *"cold chain"* that is an end-to-end temperature-controlled supply chain to transport some compounds, such as biological fluids, from one part of the workshop to another part. Of course, during this transport, extreme care needs to be taken to avoid the degradation and the contamination of the product. Single-use plastic containers such as bags are often used to transport the products. The plastic must withstand a large range of temperatures. Indeed, the plastic must not become brittle in cold temperatures to avoid failures of the containers. The containers must also be sterilized before introducing the compounds. Sterilization by a heat treatment is preferable to sterilization by gamma treatment to avoid using a radioactive source. Yet, the plastics used for some bags such as polyethylene do not withstand the high temperatures of sterilization.

Multilayer bags with layers of several polymer layers can be used but the polymers used may have different glass transition temperatures from which it results in a risk of delamination and product loss. Moreover, adhesive layers are often used to improve the adhesion between the layers which adds complexity in the preparation of the bags.

### Background art

US 2006/0246244 discloses a disposable vessel useful for the containment of biological materials and corrosive reagents made with a fluoropolymer.

Two-layers containers have been developed to address the issue of multilayer bags in the pharmaceutical industry. US 2018/0104147 discloses a single-use bag including an inner film defining the interior of the container and an outer film overlying the inner film, the inner film and the outer film being heat bonded together, the inner film being composed of perfluoroalkoxyalkane (PFA) and the outer film being composed of polytetrafluoroethylene (PTFE).

### Technical problem to be solved

There is still a need for a resistant container for storing products of high value without contamination from the container. The present invention aims at solving this technical problem.

The invention addresses more particularly the problem to find a resistant and flexible solution which can be used in a large range of temperatures and can be sterilized by irradiation.

### Brief disclosure of the invention

The invention is set out in the appended set of claims.

The invention relates to a container as defined in claims 1-19.

The invention also relates to the use of a composite film as defined in claims 20-21.

These subject-matters are now defined in more details below.

### General definitions

The proportion of recurring units in a polymer are given in mol.% relative to the total number of moles of recurring units in the polymer.

When numerical ranges are indicated, range ends are included.

### Figure

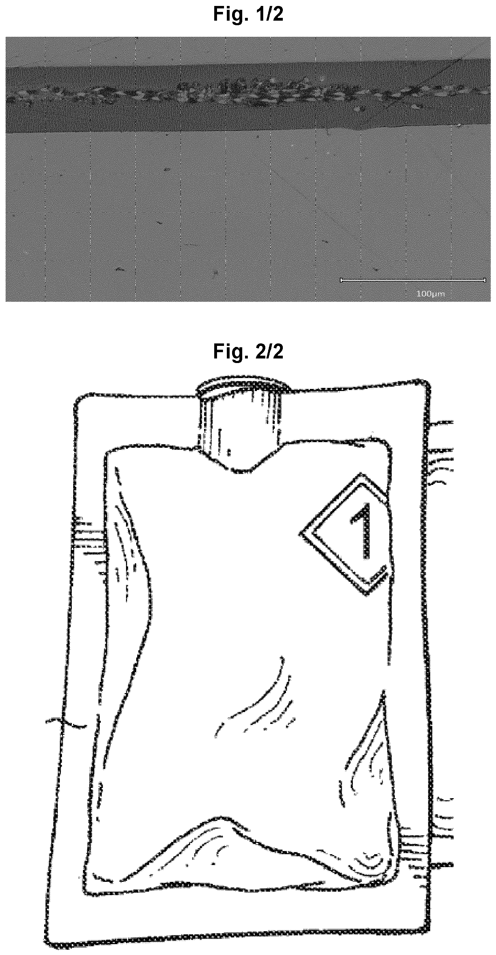

**Fig. 1/2** represents an image obtained with a microscope of a composite film (CF) according to the invention. One can clearly see the glass fiber fabric (F) and the two layers (L1) (L2) of fluoropolymer.
**Fig. 2/2** represents an example of container according to embodiment (E1).

### Disclosure of the invention

The invention relates to a container, optionally comprising a product, wherein the container comprises at least one composite film (CF) which comprises a fiber fabric (F) in between two layers (L1) and (L2) made of or comprising at least one fluoropolymer (FP) comprising recurring units (R_{TFE}) of formula -(CF₂-CF₂)-.

The product is in contact with at least a part of either layer (L1) or (L2). As the fluoropolymer (FP) is chemically inert, the container is adapted to the storage of corrosive or sensitive products. In addition, the fluoropolymer (FP) may be such that it also contains a low proportion of low molecular weight molecules, so that a sensitive product can be stored safely without the risk of being contaminated by the migration of low molecular weight molecules from the fluoropolymer (FP).

The container of the invention comprises at least one composite film (CF). Either layer (L1) or (L2) of the composite film (CF) is to be in contact with the contained product.

The container may be in a variety of forms. The container may be in the form of a pouch, a bottle, a bag or a tank. The container may comprise several zones in which the product can be stored.

The composite film may define the interior of the container. The invention also encompasses the situation wherein the container comprises several zones able to contain a product. In this situation, the composite film defines at least one of said zones.

The container can be used to store a large variety of products. The product may be in the solid or liquid form.

For instance, the product that can be stored in the container may be a biological product. A biological product is defined as a product which is used to diagnose, prevent, treat, and cure diseases and medical conditions of humans or animals. A biological product may be selected in the group consisting of therapeutic proteins, monoclonal antibodies, vaccines and molecules having a molecular weight lower than 500 g/mol.

The product that can be stored in the container may be a chemical product, notably a chemical product to be used in the industry of preparation of wafers. The chemical product may for instance H₂O₂.

Specific embodiments illustrating the invention now provided below.

The invention also relates to the use of the composite film (CF) for the preparation of a container, notably as described herein.

### Embodiment (E1)

According to a first embodiment (E1), the layer (L1) (or (L2)) of the composite film (CF) defines the interior of the container and layer (L2) (or respectively (L1)) of the composite film (CF) defines the exterior of the container.

Such a container may be prepared by bonding (eg by heat bonding) together the edges of two composite films (CF). Heat bonding involves welding overlapping lengths of the composite film (CF) together by applying heat to the overlap. The bonding is achieved by heating the overlapped surfaces, usually under pressure.

According to an embodiment, all edges are bonded together such as to form a closed structure. According to another embodiment, only a part of the edges are bonded together so as to leave the possibility to add at least one passage by which the product can pass through.

As the thickness of the composite film (CF) may be low, the container according to this embodiment (E1) can be rendered flexible. This embodiment is therefore adapted for the preparation of single-use bags, notably for storing biological products.

The container once made can be sterilized. Sterilization can be carried out on the container by exposing it to an ionizing radiation. The ionizing radiation is preferably gamma radiation. The effective dosage of radiation and duration of the exposure to the radiation are selected to achieve the targetted level of sterilization. Typically, such dosage is in the range of about 25 to 40 kGy.

### Embodiment (E2)

According to a second embodiment (E2), the container comprises the composite film (CF) as the inner wall and at least one additional layer as the outer wall of the container comprising at least one thermoplastic polymer. The thermoplastic polymer may be selected in the group consisting of polyethylene (*eg* High Density PolyEthylene or Low Density PolyEthylene), polypropylene, polyamide (*eg* polyamide 66), polystyrene and fluoropolymer (*eg* a (co)polymer comprising the recurring units of vinylidene fluoride or TFE).

The thickness of the outer wall is chosen in order to have the right combination of mechanical properties such as the rigidity of the container or the resistance to an external impact. It is also noted that the selection of the appropriate thermoplastic polymer makes it also possible to control the overall price of the container.

A function of the outer wall is to provide rigidity to the container. The thickness of the outer wall is thus adapted to the targetted rigidity.

The composite film (CF) may be directly in contact *e.g*. via the layer (L1) or (L2), with the outer wall of the container.

Yet, it is also possible to have at least one additional layer between the inner wall and the outer wall to provide additional properties to the container. For instance, the at least additional layer may be a barrier layer to avoid the diffusion of molecules from outside inside the container.

The at least additional layer may also be an adhesive layer to improve the adhesion of the two layers with which the adhesive layer is in direct contact. For instance, the structure of the container may be the following: (outer wall) / (adhesive layer) / layer (L1) or (L2) of the composite film (CF) / fiber fabric / (L2) or (L1) of the composite film (CF). The adhesive layer may comprise or be made of a thermoplastic polymer having functional polar groups such as COOH, anhydride, OH, amine, amide groups.

An example will now be disclosed of a container having this structure: (outer wall made of a thermoplastic polymer such as polyethylene e.g. HDPE) / (adhesive layer comprising or made of a copolymer of ethylene, maleic anhydride and optionally a C1-C6-alkyl acrylate) / layer (L1) (or (L2)) of the composite film (CF) / fiber fabric / (L2) (or (L1)) of the composite film (CF). The adhesion between layer (L1) (or (L2)) and the adhesive layer may be improved if layer (L1) (or (L2)) also comprises polar functional groups. For instance, layer (L1) (or (L2)) may comprise at least the fluoropolymer (FP) as previously disclosed and an additional fluoropolymer having polar functional groups. An example of such a combination is for instance disclosed in WO 2007/110529.

### The composite film (CF)

The composite film (CF) comprises a fiber fabric (F) in between two layers (L1) and (L2) made of or comprising at least one fluoropolymer (FP) comprising recurring units (R_{TFE}) of formula -(CF₂-CF₂)-.

The two layers (L1) and (L2) may be made or comprise the same fluoropolymer (FP) but the invention also covers a composite film (CF) wherein the two layers (L1) and (L2) are made of or comprise two fluoropolymers (FP) that differ from one another by their chemical structure and/or by their physicochemical properties.

The overall thickness of the composite film (CF) may be between 10.0 µm and 150.0 µm. The thickness may more particularly be between 15.0 and 100.0 µm.

Because of the combination of the fiber fabric (F) and the fluoropolymer (FP), the composite film (CF) exhibits certain physico-chemical properties. The composite film (CF) may exhibit a tensile strength (along 0 degree of fiber direction) of at least 70.0 MPa, preferably at least 100.0 MPa, preferably at least 140.0 MPa. The tensile strength is measured according ASTM D3039. The tensile strength is usually at most 200.0 MPa.

### The fluoropolymer (FP)

The fluoropolymer (FP) used in layer (L1) and/or (L2) of the composite film (CF) comprises the recurring units (R_{TFE}) derived from tetrafluoroethylene (TFE).

The fluoropolymer (FP) may more particularly be selected in the group consisting of PTFE and copolymers of TFE as defined below.

The fluoropolymer (FP) is advantageous since it can withstand a large range of temperatures and can be sterilized by irradiation.

### PTFE

The fluoropolymer (FP) may be a PTFE. In the context of the present invention, PTFE denotes a polymer comprising at least 98.5 mol.% of recurring units (R_{TFE}). The proportion of the recurring units is expressed in mol.% and is based on the total number of moles of the recurring units in the fluoropolymer (FP).

The PTFE may also comprise up to 1.5 mol.% of recurring units derived from at least one ethylenically unsaturated fluorinated monomer distinct from TFE (monomer M). This monomer (M) may more particularly be selected from the group consisting of:
- perfluoroalkylvinylethers of formula CF₂=CFOR_{f} wherein R_{f} is a C₁-C₆ perfluoroalkyl group, preferably a C₁-C₃ perfluoroalkyl group;
- perfluoro-oxyalkylvinylethers of formula CF₂=CFOX₀ wherein X₀ is a C₁-C₁₂ perfluorooxyalkyl group comprising one or more ether groups, such as perfluoro-2-propoxy-propyl group;
- C₃-C₈ perfluoroolefins, such as hexafluoropropene (HFP); and
- perfluorodioxoles of formula (I): 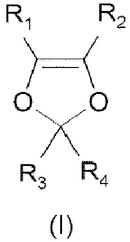 wherein R₁, R₂, R₃ and R₄, equal to or different from each other, are independently selected from the group consisting of -F, a C₁-C₆ fluoroalkyl group, optionally comprising one or more oxygen atoms, and a C₁-C₆ fluoroalkoxy group, optionally comprising one or more oxygen atoms.

The monomer (M) may more particularly be selected in the group consisting of perfluoroalkylvinyl ethers of formula CF₂=CFOR_{f} wherein R_{f} is a C₁-C₆ perfluoroalkyl group, preferably a C₁-C₃ perfluoroalkyl group.

The proportion of recurring units (R_{TFE}) may more particularly be at least 99.0 mol.%, at least 99.5 mol.% or at least 99.9 mol.%. The proportion of the recurring units derived from the ethylenically unsaturated fluorinated monomer distinct from TFE may then be respectively up to 1.0 mol.%, up to 0.5 mol.% or up to 0.1 mol.%.

According to an embodiment, the recurring units of the PTFE consist in the recurring units (R_{TFE}) and the recurring units of at least one ethylenically unsaturated fluorinated monomer distinct from TFE.

According to a preferred embodiment, the PTFE comprises 100 mol.% of recurring units (R_{TFE}).

According to an embodiment, the PTFE may be provided in the form of powders, which may be obtained by irradiation of standard high molecular weight PTFE or modified PTFE, and which are generally known for possessing a considerably lower molecular weight than the typical molecular weight of standard high molecular weight/high melt viscosity PTFEs/modified PTFEs. This enables the micropowders of PTFEs and/or modified PTFE to be by themselves melt-flowable.

Preferably, the fluoropolymer (FP) exhibits a melt viscosity of at most 1.5×10³ Pa.s, as measured according to ASTM D3835 at 372°C and 1000 s⁻¹ using a Hastelloy die of 1 mm × 10 mm. The melt viscosity can be at most 1.4×10³ Pa.s, at most 1.3×10³ Pa.s, at most 1.0×10³ Pa.s or at most 0.8×10³ Pa.s.

### Copolymers of TFE

The polymer (FP) may be a copolymer of TFE comprising recurring units (R_{TFE}) and recurring units derived from at least one monomer (M), the proportion of which being strictly higher (>) than 1.5 mol.% and at most 30.0 mol.%, wherein the monomer (M) is selected from the group consisting of:
- perfluoroalkylvinylethers of formula CF₂=CFOR_{f} wherein R_{f} is a C₁-C₆ perfluoroalkyl group, preferably a C₁-C₃ perfluoroalkyl group;
- perfluoro-oxyalkylvinylethers of formula CF₂=CFOX₀ wherein X₀ is a C₁-C₁₂ perfluorooxyalkyl group comprising one or more ether groups, such as perfluoro-2-propoxy-propyl group;
- C₃-C₈ perfluoroolefins, such as hexafluoropropene (HFP); and
- perfluorodioxoles of formula (I): 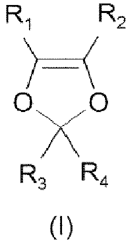 wherein R₁, R₂, R₃ and R₄, equal to or different from each other, are independently selected from the group consisting of -F, a C₁-C₆ fluoroalkyl group, optionally comprising one or more oxygen atoms, and a C₁-C₆ fluoroalkoxy group, optionally comprising one or more oxygen atoms.

The proportion of the recurring units derived from monomer (M) may be at least 3.0 mol.% or at least 5.0 mol. % or at least 7.0 mol.%.

The polymer (FP) may advantageously be a copolymer of TFE comprising:
- between 3.0 and 6.0 mol. % of recurring units derived from at least one perfluoroalkylvinylether selected in the group consisting of perfluoromethylvinylether, perfluoroethylvinylether and perfluoropropylvinylether, and
- between 94.0 and 97.0 mol. % of recurring units (R_{TFE}).

The polymer (FP) may advantageously be a copolymer of TFE comprising:
- between 3.0 and 6.0 mol. % of recurring units derived from perfluoromethylvinylether or perfluoroethylvinylether or perfluoropropylvinylether, and
- between 94.0 and 97.0 mol. % of recurring units (R_{TFE}).

The perfluoroalkylvinylether may be perfluoromethylvinylether. The perfluoroalkylvinylether may be prefluoroethylvinylether. The perfluoroalkylvinylether may be perfluoropropylvinylether.

The fluoropolymer (FP) may have a melt viscosity of at most 1.8×10³ Pa.s, as measured according to ASTM D3835 at 372°C and 100 s⁻¹ using a Hastelloy die of 1 mm × 10 mm, for example at most 1.7×10³ Pa.s, at most 1.6×10³ Pa.s, at most 1.5×10³ Pa.s or at most 1.4×10³ Pa.s.

Non-limitative examples of suitable fluoropolymer (FP) include notably those commercially available under the trademark name HYFLON^{®} PFA P and M series and HYFLON^{®} MFA from Solvay Specialty Polymers Italy S.p.A.

### Fiber fabric (F)

The fiber fabric (F) used in the composite film may be an aramid fabric, a glass fiber fabric or a quartz fabric.

The thickness of the fiber fabric (F) may be between 0.01 mm and 0.09 mm. The thickness may more particularly be between 0.02 mm and 0.07 mm.

The fiber fabric (F) may exhibit an average areal weight in g/m² (or gsm) comprised between 10.0 g/m² and 100.0 g/m². The average areal weight may be more particularly between 15.0 g/m² and 75.0 g/m² or between 15.0 g/m² and 60.0 g/m². The average areal weight may be between 15.0 and 35.0 g/m².

The fiber fabric (F) is more particularly a glass fiber fabric.

The glass fibers are made of SiO₂ and other oxides such as Al₂O₃, MgO or CaO.

The glass fiber fabric is a fabric obtained by weaving glass fiber yarns. The glass strand used as the glass fiber fabric is usually formed by pulling and aligning glass fibers having a diameter of about several microns in units of several hundred. The characteristics of the glass fiber fabric are determined by fiber properties, warp and weft density, yarn structure and texture. The warp and weft density is determined by the yarn structure and texture. The warp and weft density plus the yarn structure determines the physical properties of the fabric, such as weight, thickness and breaking strength. The basic texture is plain, twill, satin, rib and basket. The type and configuration of the glass fiber fabric are not particularly limited.

### Preparation of the composite film (CF)

The composite film (CF) may be prepared by the method comprising the following steps:
a) applying the fluoropolymer (FP) on at least one surface of the fiber fabric (F) to prepare a structure (S) wherein the fluoropolymer is in contact with the fibers of the fiber fabric (F);
b) heating the fluoropolymer (FP) at a temperature T > Tm, wherein Tm is the melting temperature of the fluoropolymer (FP) and applying a pressure P on the structure (S).

The method may be performed according to two embodiments. According to an embodiment, step a) is performed by applying the fluoropolymer (FP) on one surface of the fiber fabric (F) before performing step b). Steps a) and b) are then repeated by applying the fluoropolymer (FP) on the other surface of the fiber fabric (F). This embodiment may notably be preferred when the two layers (L1) and (L2) are made or comprise two fluoropolymers (FP) that differ from one another by their chemical structure and/or by their physicochemical properties. For instance, if the two layers (L1) and (L2) are made or comprise two fluoropolymers (FP) that exhibit two different Tm, step b) may then require specific conditions of temperature and/or pressure.

According to another embodiment, step a) is performed by applying the fluoropolymer (FP) on the two surfaces of the fiber fabric (F) before performing step b).

After step(s) b), the layer (L1) and/or (L2) are formed. A protective film may be applied on at least one of such formed layers. The function of the protective film is to avoid the contamination of such formed layer(s). It is removed before use of the composite film (CF).

At the temperature and pressure referenced above, the fluoropolymer (FP) undergoes a phase transition, typically melting, enabling it to bond securely to the fiber fabric.

In step a), the fluoropolymer (FP) may be in the form of powder or in the form of an aqueous based dispersion, eg. dispersion or emulsion. The fluoropolymer (FP) is advantageously in the form of powder to avoid the use of any liquid that needs to be taken out during step b).

In a preferred embodiment, the fluoropolymer (FP) is in the form of a powder and is applied onto both surfaces of the fiber fabric.

The fluoropolymer (FP) is advantageously in the form of powder characterized by an average particle size d50, determined by laser light diffraction (for instance using a laser diffraction particle size LS^{™} 13 320 MW - Beckman Coulter instrument) according to ISO 13320, of at most 25.0 µm, for example at most 22.0 µm or at most 20.0 µm. Lower boundary for d50 is not particularly limited. It is nevertheless understood that to the sake of convenience in handling the powder, the d50 is generally at least 0.5 µm, preferably at least 1.0 µm. d50 may more particularly between 2.0 µm and 15.0 µm, preferably of between 2.5 µm and 12.0 µm.

An example of appropriate powder is POLYMISTO PTFE micronized powder commercially available from Solvay Specialty Polymers USA, LLC.

Step b) is conveniently performed with a hot press.

If a molding press is used, a release film between the external surface of structure S and the platen of the press may be used, so as to avoid any sticking of the external surface to the platen occurs. Any release film, which does not interfere with or alter the characteristics of the composite film, is suitable. The release film can be a polyimide, or a release coated metal foil such as aluminum, for example.

The pressure and the temperature applied in step b) depend upon the type of fluoropolymer employed, the form in which it is applied on the surface and the fiber fabric employed.

The conditions of the experimental section may be followed for the preparation of the composite film (CF).

### Experimental section

Fluoroolymer (FP): Hyflon PFA P7010 commercialized by Solvay. Melting temperature Tm = 305°C.

Glass fiber fabric (F):
- glass fiber fabric 104 from BGF Industries;
- glass fiber fabric 106 from BGF Industries;
- glass fiber fabric 1080 from BGF Industries.

### Properties of the fabrics used

| **Fiber fabric used** | **Aeral weight** |
|---|---|
| 104 | 20 gsm |
| 106 | 25 gsm |
| 1080 | 48 gsm |

### Recipe for the preparation of the composite films

The fluoropolymer was applied in the powder form on both faces of the glass fiber fabrics (FP / glass fabric / FP). The obtained structures were heated at 300°C for 10 minutes in a press under a pressure of 17 bar. The structures were then removed from the press and left to cool to ambient temperature.

## Claims

1. Container, optionally comprising a product, comprising at least one composite film (CF) which comprises a fiber fabric (F) in between two layers (L1) and (L2) made of or comprising at least one fluoropolymer (FP) comprising recurring units (R_{TFE}) of formula -(CF₂-CF₂)-.

2. Container according to claim 1, wherein the product is in contact with at least a part of either layer (L1) or (L2).

3. Container according to any one of claims 1-2, wherein the composite film defines the interior of the container.

4. Container according to any one of claims 1-2, wherein the composite film defines at least one zone of the container where the product can be contained.

5. Container according to any one of the preceding claims, wherein the container is in the form of a pouch, a bottle, a bag or a tank.

6. Container according to any one of the preceding claims, wherein the thickness of the composite film (CF) is between 10.0 and 150 µm, more particularly between 15.0 and 100.0 µm.

7. Container according to any one of the preceding claims, wherein the composite film (CF) exhibits a tensile strength (along 0 degree of fiber direction and measured according to ASTM D3039) of at least 70.0 MPa, preferably at least 100.0 MPa, preferably at least 140.0 MPa.

8. Container according to any one of the preceding claims, wherein the fluoropolymer (FP) of layer (L1) and/or (L2) is selected in the group consisting of PTFE and copolymers of TFE wherein:
- the term PTFE denotes a polymer comprising at least 98.5 mol.% of recurring units (R_{TFE});
- the term copolymer of TFE denotes a polymer comprising recurring units (R_{TFE}) and recurring units derived from at least one monomer (M), the proportion of which being strictly higher (>) than 1.5 mol.% and at most 30.0 mol.%, wherein the monomer (M) is selected from the group consisting of:
- perfluoroalkylvinylethers of formula CF₂=CFOR_{f} wherein R_{f} is a C₁-C₆ perfluoroalkyl group, preferably a C₁-C₃ perfluoroalkyl group;
- perfluoro-oxyalkylvinylethers of formula CF₂=CFOX₀ wherein X₀ is a C₁-C₁₂ perfluorooxyalkyl group comprising one or more ether groups, such as perfluoro-2-propoxy-propyl group;
- C₃-C₈ perfluoroolefins, such as hexafluoropropene (HFP); and
- perfluorodioxoles of formula (I): 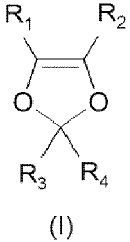
wherein R₁, R₂, R₃ and R₄, equal to or different from each other, are independently selected from the group consisting of -F, a C₁-C₆ fluoroalkyl group, optionally comprising one or more oxygen atoms, and a C₁-C₆ fluoroalkoxy group, optionally comprising one or more oxygen atoms,
the proportion of the recurring units being expressed in mol.% and is based on the total number of moles in the fluoropolymer.

9. Container according to claim 8, wherein the PTFE comprises up to 1.5 mol.% of recurring units derived from at least one monomer M.

10. Container according to any one of the preceding claims, wherein the fluoropolymer (FP) of layer (L1) and/or (L2) is a copolymer of TFE comprising:
- between 3.0 and 6.0 mol. % of recurring units derived from at least one perfluoroalkylvinylether which is perfluoromethylvinylether, perfluoroethylvinylether or perfluoropropylvinylether, and
- between 94.0 and 97.0 mol. % of recurring units (R_{TFE}).

11. Container according to any one of the preceding claims, wherein the fluoropolymer (FP) of layer (L1) and/or (L2) is has a melt viscosity of at most 1.8×10³ Pa.s, as measured according to ASTM D3835 at 372°C and 100 s⁻¹ using a Hastelloy die of 1 mm × 10 mm.

12. Container according to any one of the preceding claims wherein the fiber fabric (F) is a glass fiber fabric.

13. Container according to any one of the preceding claims, wherein the fiber fabric (F) exhibits an average areal weight comprised between 10.0 g/m² and 100.0 g/m².

14. Container according to claim 12, wherein the fiber fabric (F) exhibits an average areal weight comprised between 15.0 g/m² and 35.0 g/m².

15. Container according to any one of the preceding claims, wherein the layer (L1) (or (L2)) of the composite film (CF) defines the interior of the container and layer (L2) (or (L1)) of the composite film (CF) defines the exterior of the container.

16. Container according to any one of claims 1 to 14, comprising the composite film (CF) as the inner wall and at least one additional layer as the outer wall of the container comprising at least one thermoplastic polymer.

17. Container according to any one of the preceding claims containing a product in the solid or in the liquid form.

18. Container according to claim 17, wherein the product is a biological product.

19. Container according to claim 17, wherein the product is a chemical product.

20. Use of a composite film (CF) comprising a fiber fabric (F) in between two layers (L1) and (L2) made of or comprising at least one fluoropolymer (FP) comprising recurring units (R_{TFE}) of formula -(CF₂-CF₂)- for the preparation of a container.

21. Use according to claim 20 wherein the fiber fabric (F) is a glass fiber fabric.

22. Use according to claims 20 or 21 wherein the fluoropolymer (FP) of layer (L1) and/or (L2) is according to one of claims 8 to 11.
